# EUROPEAN PATENT APPLICATION

(11) **EP 4 792 807 A1**
(43) Date of publication of application: **19.08.2026**
(21) Application number: 24877194.1
(22) Date of filing: 09.10.2024
(51) Int. Cl.: A61K 9/51, A61K 9/107, A61K 9/113, A61K 9/127, A61K 31/70, A61K 31/7088, A61K 39/395, A61K 45/00, A61K 48/00

(54) **CARRIER CAPABLE OF TARGETING CELL AT SUBSET LEVEL AND METHOD FOR PRODUCING SAME**

(30) Priority: 11.10.2023 JP 2023175890
(71) Applicant: National University Corporation Kanazawa University, Ishikawa 920-1192 (JP)
(72) Inventor: NAKAMURA, Takashi, Sapporo-shi, Hokkaido 060-0808 (JP); HARASHIMA, Hideyoshi, Sapporo-shi, Hokkaido 060-0808 (JP); IKENAGA, Tokikazu, Sapporo-shi, Hokkaido 060-0808 (JP); SHIMA, Natsumi, Sapporo-shi, Hokkaido 060-0808 (JP)
(74) Representative: Kraus & Lederer PartGmbB
(86) International application number: PCT/JP2024/036074
(87) International publication number: WO 2025/079599

(57) **Abstract**

A problem to be solved by the present invention is to provide a carrier capable of being selectively delivered to cells at a subset level, which has not yet been realized by conventional art, and to provide a method for producing the carrier. The present invention relates to a carrier for selective delivery to a target cell having multiple types of target receptors on its surface, the carrier having multiple types of ligands, wherein each type of ligand binds to one of the multiple types of target receptors, wherein the carrier is taken up by the target cell that has all the multiple types of target receptors, but is not taken up by a cell that lacks at least one of the multiple types of target receptors; a method for producing the carrier; and the like.

## Description

### TECHNICAL FIELD

The present invention relates to a carrier capable of being delivered specifically to target cells, in particular, a subset of cells, and to a method for producing the carrier.

### BACKGROUND ART

DDS (drug delivery system) technology using liposomes or lipid nanoparticles (LNPs) has been applied as medicines, such as, for example, DOXIL, which is an anticancer drug encapsulated in liposomes; onpattro, which is siRNA-loaded LNPs; or mRNA vaccines, which are mRNA-loaded LNPs. However, the tissue or cell selectivity of DDS technology is insufficient in targeting tissues other than the liver and spleen, which are highly susceptible to the accumulation of nanoparticles. Such selectivity has been a critical issue in the current development of DDS (see, e.g., Patent Literature 1, etc.).

To achieve cell targeting, research and development of DDS technology have been conducted by modifying nanoparticles with ligand molecules, such as antibodies or targeting peptides. As an example of such DDS technology, a dual-targeting strategy using multiple ligands has been investigated.

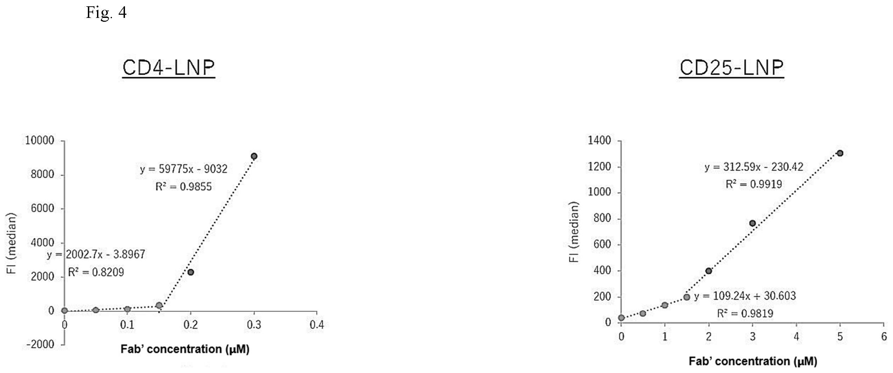

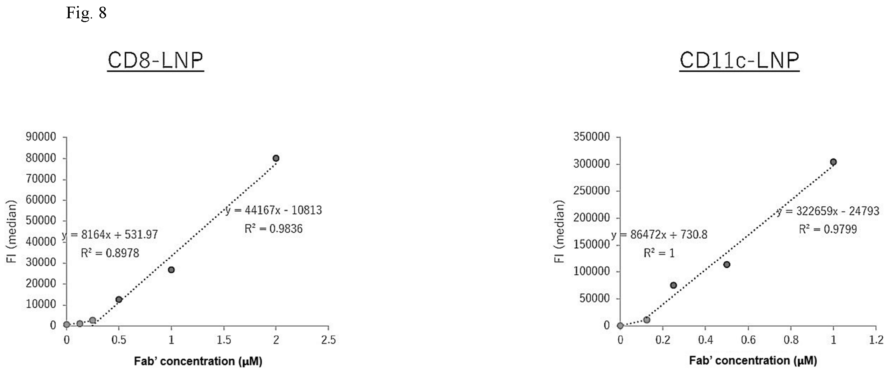

For example, Non-Patent Literature 1 has confirmed that the amount of uptake of CESP (sali-entrapped lipid-polymer nanoparticles labeled with CD133 and EGFR aptamers) by EGFR+/CD133+ cells is greater than that of nanoparticles labeled with a single aptamer (CSP or ESP) (Figure 4 and Figure 8, etc.).

Non-Patent Literature 2 has demonstrated that when M2NPs (M2-like TAM dual-targeting nanoparticles) using, as a ligand, a fusion peptide consisting of α-peptide linked with M2pep via a GSG linker are allowed to be taken up by M2 macrophages, the M2NPs exhibit a synergistic effect as compared with nanoparticles labeled with a single aptamer (ABSTRACT).

The technologies disclosed in these references are intended to increase the amount of uptake of the nanoparticles by cells, but do not realize the targeting of a narrower range of cell populations (a subset of cells).

In recent years, the detailed cellular diversity and hierarchy discovered because of the development of single-cell omics analysis technology have revealed the detailed mechanisms of diseases and uncovered the complicated involvement of various subsets of cells.

However, almost all the conventional DDS technologies are designed to identify a single target molecule to improve the affinity of nanoparticles. No targeting technology capable of selectively delivering a drug to cells at a subset level has not yet been developed.

Inhibition of gene expression by siRNAs is an established technology, and testing and research have been conducted worldwide using various siRNAs, including commercially available siRNAs (see, e.g., Non-Patent Literature 3).

### CITATION LIST

### PATENT LITERATURE

Patent Literature 1: JP 2022-111798 A

### NON-PATENT LITERATURE

Non-Patent Literature 1: Master F, et al., "Targeted salinomycin delivery with EGFR and CD133 aptamers based dual-ligand lipid-polymer nanoparticles to both osteosarcoma cells and cancer stem cells", Nanomedicine, 14, 7, 2115-2127 (2018).
Non-Patent Literature 2: Qian Y, et al., "Molecular-Targeted Immunotherapeutic Strategy for Melanoma via Dual-Targeting Nanoparticles Delivering Small Interfering RNA to Tumor-Associated Macrophages", ACS Nano, 26, 11, 9, 9536-9549 (2017).
Non-Patent Literature 3: Nakamura T, et al., "Small-sized, stable lipid nanoparticle for the efficient delivery of siRNA to human immune cell lines", Sci Rep 6: 37849, 2016.

### SUMMARY OF INVENTION

### TECHNICAL PROBLEM

The inventors have set the goal to develop a carrier capable of targeting cells at a subset level because once subset-level targeting of cells involved in the mechanism of a disease is realized, future drug development will be greatly accelerated.

Therefore, a problem to be solved by the present invention is to provide a carrier capable of being selectively delivered to cells at a subset level, which has not yet been realized by the conventional art, and to provide a method for producing the carrier.

### SOLUTION TO PROBLEM

The inventors conducted extensive studies to solve the above problems and found that a carrier of which the surface is modified with controlled densities of multiple types of ligands exhibits selective affinity for a subset of cells defined by multiple target molecules. The inventors conducted further studies and completed the invention.

Therefore, the present invention relates to the following.
[1] A carrier for selective delivery to a target cell having multiple types of target receptors on its surface, the carrier having multiple types of ligands, wherein each type of ligand binds to one of the multiple types of target receptors, wherein the carrier is taken up by the target cell that has all the multiple types of target receptors, but is not taken up by a cell that lacks at least one of the multiple types of target receptors.
[2] The carrier according to the above [1], wherein each type of ligand modifies the carrier at a modification concentration at which
   (a) any single type of ligand is insufficient to allow the carrier to be taken up by the target cell, but
   (b) all the multiple types of ligands together allow the carrier to be taken up by the target cell.
[3] The carrier according to the above [1] or [2], wherein the multiple types of target receptors are two to five types of receptors.
[4] The carrier according to any one of the above [1] to [3], wherein the receptors are antigens, and the ligands are antibodies or antibody fragments.
[5] The carrier according to any one of the above [1] to [4], wherein the carrier is a lipid nanoparticle, a polymer nanoparticle, a unilamellar liposome, a multilamellar liposome, an O/W type emulsion, a W/O/W type emulsion, a spherical micelle, a wormlike micelle, a lamellar structure, or a mitochondrion.
[6] The carrier according to any one of the above [1] to [5], wherein the target cell is a subset of immune cells, a subset of cancer cells, or a subset of vascular endothelial cells.
[7] The carrier according to the above [6], wherein the subset of immune cells is a subset of T cells, a subset of NK/NKT cells, or a subset of dendritic cells.
[8] The carrier according to any one of the above [1] to [7], wherein the carrier comprises a nucleic acid, a sugar, a medium molecular weight compound, a low molecular weight compound, or a metal compound.
[9] A method for producing a carrier for selective delivery to a target cell having multiple types of target receptors on its surface, the method comprising
   (1) determining a modification concentration of each of multiple types of ligands, wherein each type of ligand binds to one of the multiple types of target receptors, wherein the modification concentration of each type of ligand is a concentration at which it is demonstrated that
      (a) any single type of ligand is insufficient to allow the carrier to be taken up by the target cell, but
      (b) all the multiple types of ligands together allow the carrier to be taken up by the target cell, and
   (2) modifying the carrier with the multiple types of ligands each at the determined modification concentration.
[10] The production method according to the above [9], wherein the determining the modification concentration of each of the multiple types of ligands is performed by plotting an amount of uptake of the carrier by the target cell against concentrations of each type of ligand, and setting the modification concentration of each type of ligand to 0.4-fold to 2.5-fold the concentration at a point of intersection of a first regression line with a gentle slope drawn from a flat plot in which the amount of uptake of the carrier by the target cell gradually increases or remains unchanged and a second regression line with a steep slope drawn from a plot in which the amount of uptake of the carrier by the target cell rapidly increases.
[11] A carrier for selective delivery to a target cell having multiple types of target receptors on its surface produced by the production method according to the above [9] or [10].
[12] The carrier according to any one of the above [1] to [8], wherein the carrier encapsulates a gene-expression inhibitor, and the carrier is used for inhibition of gene expression.
[13] The carrier according to the above [12], wherein the gene expression inhibitor is an siRNA.
[14] The carrier according to the above [12], wherein the carrier is used for achieving remission or treatment of a disease caused by overexpression of a gene.
[15] The carrier according to the above [14], wherein the disease caused by overexpression of a gene is a cancer or an autoimmune disease.

### ADVANTAGEOUS EFFECTS OF INVENTION

In the conventional art, nanoparticles are taken up by all cells expressing target receptors, regardless of the subset of cells. In contrast, in the present invention, the modification densities of multiple types of ligands on a carrier, such as nanoparticles, are controlled so that the carrier is taken up by a target cell only after the multiple types of ligands bind to their respective multiple types of receptors. In this manner, the carrier exhibits the excellent effect of selectively recognizing a subset of cells defined by the multiple types of surface receptors, thereby being able to be delivered to the subset of cells.

Even when cells classified as the same type exist, the carrier of the present invention enables selective delivery of the carrier itself or a component encapsulated in the carrier to a subset of cells classified by a combination of receptors that reside on the surface of cells. Therefore, the present invention provides a carrier that can be made applicable to a wide variety of fields by appropriately labelling the carrier itself or appropriately selecting a cargo within the carrier so that, for example, the carrier is formulated into an advanced medicine using DDS technology, or the carrier is used to detect a subset of cells in the field of research and investigation.

The carrier of the present invention can contain a gene-expression inhibitor to allow the gene-expression inhibitor to specifically exert its effects on a target cell population at a subset level, thereby effectively inhibiting gene expression. In particular, the inhibition of gene expression by the carrier is expected to achieve efficient remission or treatment of a disease.

### BRIEF DESCRIPTION OF DRAWINGS

Fig. 1 is an explanatory diagram showing an overview of the present invention.
Figs. 2A-C show the preparation of Fab' fragments from antibodies. Fig. 2A is a schematic showing the preparation steps. Fig. 2B shows the electrophoresis of purified Fab' fragments purified using a size exclusion column. Fig. 2C shows the percent recovery of the purified Fab' fragments.
Fig. 3 is a diagram showing the preparation of Fab'-modified LNPs.
Fig. 4 is a graph showing the evaluation of cellular binding of CD4-LNPs and CD25-LNPs (in vitro).
Fig. 5 is a graph showing the evaluation of cellular binding of CD4/CD25-LNPs (in vitro).
Fig. 6 is a graph showing the evaluation of cellular binding of NK1.1-LNPs and CD27-LNPs (in vitro).
Fig. 7 is a graph showing the evaluation of cellular binding of NK1.1/CD27-LNPs (in vitro).
Fig. 8 is a graph showing the evaluation of cellular binding of CD8-LNPs and CD11c-LNPs (in vitro).
Fig. 9 is a graph showing the evaluation of cellular binding of CD8/CD11c-LNPs (in vitro).
Fig. 10 shows a graph showing the influence of the number of LNP particles on the selectivity of LNPs (in vitro).
Fig. 11 is a graph showing the evaluation of cellular binding of CD4/CD25-LNPs (in vivo).
Fig. 12 is a graph showing the cell selectivity of DiD-modified LNPs.
Fig. 13 is a graph showing the inhibitory effect of LNPs on the expression of a target gene.
Fig. 14 is a graph showing the inhibitory effect of LNPs on the expression of a target gene (Foxp3).

### DESCRIPTION OF EMBODIMENTS

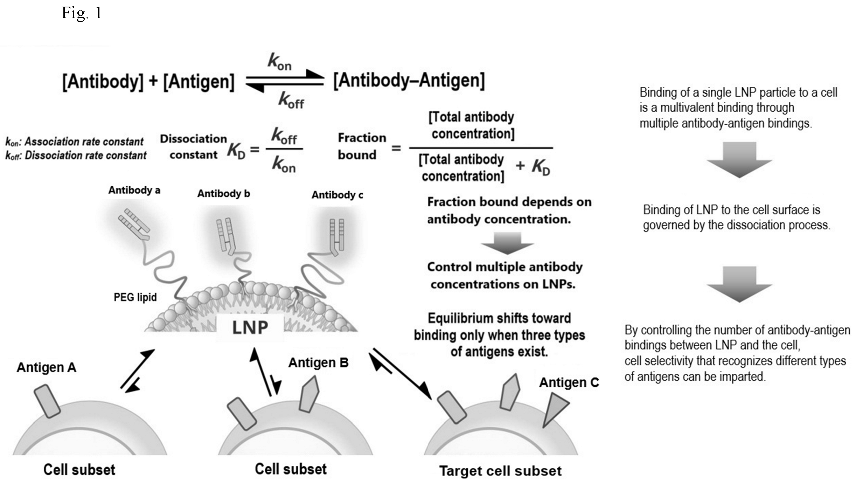

The following is an overview of the present invention, with reference to Fig.1, presented as an example where the carrier is a nanoparticle.

Binding of a single nanoparticle to a cell is a multivalent binding through multiple antibody-antigen bindings. The binding of the nanoparticle to the cell surface is governed by the dissociation process (Fig. 1). Based on this, we control the number of antibody-antigen bindings between the nanoparticle and the cell, thereby imparting cell selectivity that recognizes different types of antigens. For example, we modify the surface of nanoparticles with three types of antibodies, i.e., antibody a, antibody b, and antibody c, with control over the densities of the antibodies (Fig. 1). If there are several types of subsets of cells, for example, cells expressing antigen A only, cells expressing antigens A and B, and cells expressing antigens A, B and C, the equilibrium shifts toward binding only when the three types of antigens exist on the cell, thereby enabling the targeting of the subset of cells. In other words, in our method, the modification concentration at which only a single type of antibody is insufficient to allow nanoparticles to bind to a given cell type is first determined for each of multiple types of antibodies. Then, according to the determined concentrations, the modification densities of the multiple types of antibodies on the surface of the nanoparticles are controlled, and the nanoparticles are modified with the multiple types of antibodies at the modification densities. In this way, the equilibrium is shifted toward binding, thereby enabling the targeting of a subset of cells.

In an embodiment, the present invention relates to a carrier for selective delivery to a target cell having multiple types of target receptors on its surface. The carrier typically has multiple types of ligands, wherein each type of ligand binds to one of the multiple types of target receptors, wherein the carrier is taken up by the target cell that has all the multiple types of target receptors, but is not taken up by a cell that lacks at least one of the multiple types of target receptors.

In an embodiment of the carrier of the present invention, each type of ligand modifies the carrier at a modification concentration at which
(a) any single type of ligand is insufficient to allow the carrier to be taken up by the target cell, but
(b) all the multiple types of ligands together allow the carrier to be taken up by the target cell.

The multiple types of target receptors may be, for example, but are not limited to, two to five types of receptors.

The receptors in the present invention may be, but are not limited to, antigens, receptors, membrane proteins, sugar chains, glycolipids, or the like. In view of the versatility, availability and other advantages of the ligands such as antibodies, the receptors are preferably antigens.

The ligands herein may be selected as appropriate for the receptors to be used in combination. For example, when antigens are selected to be used as the receptors, the ligands are preferably antibodies or antibody fragments.

The antibody fragments may be, but are not limited to, any antibody fragments capable of specifically binding to the receptors. Examples of the antibody fragments include Fab, Fab', F(ab')₂, variable fragments (Fv), disulfide-stabilized Fv, single-chain variable Fv (scFv), sc(Fv)₂, diabodies, polyspecific antibodies, nanobodies, etc.

The carrier of the present invention may be, for example, but is not limited to, a lipid nanoparticle, a polymer nanoparticle, a unilamellar liposome, a multilamellar liposome, an O/W type emulsion, a W/O/W type emulsion, a spherical micelle, a wormlike micelle, or a lamellar structure. The carrier of the present invention may be any material of which the surface can be modified with the ligands, and may be, for example, a material from living organisms, such as a mitochondrion. Preferably, the carrier of the present invention is a lipid nanoparticle.

The component encapsulated in the carrier may be, but is not limited to, any substance with a size that can be accommodated in the carrier, and can include, for example, a nucleic acid, a sugar, a medium molecular weight compound, a low molecular weight compound, a metal compound, or the like.

The nucleic acid that can be encapsulated in the carrier of the present invention may be DNA, RNA, or an analogue or a derivative thereof (for example, a peptide nucleic acid (PNA), phosphorothioate DNA, etc.). The nucleic acid may be a single-stranded nucleic acid, a doublestranded nucleic acid, a linear nucleic acid, or a circular nucleic acid. The nucleic acid may contain a foreign gene to be expressed in the target cell, or may be a nucleic acid that functions to help drive the expression of a foreign gene in the target cell after being taken up by the cell. The foreign gene may be a gene that originates from the genomic DNA of the target cell, or a gene that does not originate from the genomic DNA of the target cell. Examples of such a nucleic acid include a gene expression vector containing a nucleic acid consisting of a nucleotide sequence encoding a gene of interest to be expressed. After the gene expression vector is introduced into cells, the gene expression vector may exist as an extrachromosomal gene in the cells, or may be incorporated into the genomic DNA by homologous recombination.

The gene expression vector may be, but is not limited to, a vector commonly used in gene therapy, etc. The gene expression vector may be a nucleic acid vector, such as a plasmid vector. The plasmid vector may be directly used in a circular form, or may be linearized by cleavage and then encapsulated into the carrier. The gene expression vector can be designed by a conventional method using a commonly used molecular biological tool based on the nucleotide sequence information of a gene of interest to be expressed, and produced using various known methods.

The nucleic acid may be a functional nucleic acid that regulates the expression of a target gene in the target cell. Examples of the functional nucleic acid include an antisense oligonucleotide, antisense DNA, antisense RNA, an siRNA, a microRNA, etc. The nucleic acid may be an siRNA expression vector that expresses an siRNA in the cell. The siRNA expression vector can be prepared from a commercially available siRNA expression vector, which may be modified as appropriate.

The sugar that may be encapsulated into the carrier of the present invention includes, for example, polysaccharides, glycolipids, etc.

The medium molecular weight compound (with a molecular weight of about 500 to 15000) that may be encapsulated into the carrier of the present invention includes, for example, peptides, cyclic peptides, peptide hormones, nucleic acid medicines, medium molecular medicines, etc.

The low molecular weight compound (with a molecular weight of less than 500) that may be encapsulated into the carrier of the present invention includes, for example, low molecular medicines, etc.

The metal compound that may be encapsulated into the carrier of the present invention includes, for example, gold, manganese, etc.

The target cell is not particularly limited in the present invention. The target cell in the present invention may be any cell that has multiple types of target receptors on its surface. Examples of the target cell include a subset of immune cells, a subset of cancer cells, a subset of vascular endothelial cells, etc. The subset of immune cells includes a subset of T cells, a subset of NK/NKT cells, a subset of dendritic cells, etc.

The subset of cells is identified by, for example, the expression of multiple types of receptors on the cell surface. For example, the subset of cells may be designated as CD4(+) CD25(+) cells (Treg cells) (one of subsets of T cells), NK1.1(+) CD27(+) cells (mature NK/NKT cells) (one of subsets of NK/NKT cells), CD8(+) CD11c(+) cells (CD8+ dendritic cells) (one of subsets of dendritic cells), or the like.

The modification density of each type of ligand on the surface of the carrier of the present invention, such as a nanoparticle, can be controlled by determining the modification concentration of each type of ligand. The modification concentration in the present invention refers to the concentration of a ligand solution before the ligand solution is added to a mixed solution of a lipid component and a component to be encapsulated into the carrier.

In an embodiment, the present invention relates to a method for producing a carrier for selective delivery to a target cell having multiple types of target receptors on its surface. The production method typically includes
(1) determining a modification concentration of each of multiple types of ligands, wherein each type of ligand binds to one of the multiple types of target receptors, wherein the modification concentration of each type of ligand is a concentration at which it is demonstrated that
   (a) any single type of ligand is insufficient to allow the carrier to be taken up by the target cell, but
   (b) all the multiple types of ligands together allow the carrier to be taken up by the target cell, and
(2) modifying the carrier with the multiple types of ligands each at the determined modification concentration.

In an embodiment of the production method of the present invention, the modification concentration of each type of ligand is determined by plotting an amount of uptake of the carrier by the target cell against concentrations of each type of ligand, and setting the modification concentration of each type of ligand to 0.4-fold to 2.5-fold the concentration at a point of intersection of a first regression line with a gentle slope drawn from a flat plot in which the amount of uptake of the carrier by the target cell gradually increases or remains unchanged and a second regression line with a steep slope drawn from a plot in which the amount of uptake of the carrier by the target cell rapidly increases.

An example of the specific procedure for determining the modification concentration of each type of ligand is as follows.

First, a carrier modified with a single type of ligand at various concentrations is prepared. The amount of uptake of the carrier at each ligand concentration by a target cell is determined. Then, the amount of uptake is plotted with the concentration of the ligand on the horizontal axis and the amount of uptake on the vertical axis. In the region where the concentration of the ligand is low, a plot is generated in which the amount of uptake gradually increases or remains unchanged. In the region where the concentration of the ligand is high, a plot is generated in which the amount of uptake rapidly increases. The plot in the region where the concentration of the ligand is low is separated from the plot in the region where the concentration of the ligand is high, and a regression line is drawn from each plot. Then, the concentration at a point of intersection of the two regression lines is determined: the first regression line with a gentle slope drawn from the plot in the region where the concentration of the ligand is low (i.e., a flat plot in which the amount of uptake of the carrier by the target cell gradually increases or remains unchanged), and the second regression line with a steep slope drawn from the plot in the region where the concentration of the ligand is high (i.e., a plot in which the amount of uptake of the carrier by the target cell rapidly increases). By setting the modification concentration of the ligand to 0.4-fold to 2.5-fold the concentration at the point of intersection, a carrier modified with a single type of ligand is not taken up by the target cell, or only a negligible amount of the carrier is taken up by the target cell.

By modifying the carrier with the multiple types of ligands each at the thus-determined modification concentration, the carrier is imparted with such a feature that the carrier is taken up by the target cell that has all the multiple types of receptors that bind to their respective ligands, but is not taken up by a cell that lacks at least one of the multiple types of receptors.

The carrier of the present invention can contain a gene-expression inhibitor and can be used for the inhibition of gene expression. The term "gene-expression inhibitor" refers to a molecule that acts on DNA, mRNA, or the like to inhibit the process of replication, transcription, and/or translation. The term typically refers to a molecule that contains a nucleotide sequence complementary to the nucleotide sequence of a target gene to transiently inhibit the expression of a protein encoded by the target gene. Specifically, the gene-expression inhibitor includes, but is not limited to, an siRNA, a miRNA, a ribozyme, an antisense nucleic acid, a DNA/RNA chimeric polynucleotide, etc.

The carrier of the present invention containing a gene-expression inhibitor can be used as a carrier for achieving remission or treatment of a disease caused by overexpression of a gene.

The disease caused by overexpression of a gene may include cancers, autoimmune diseases, etc. These diseases have been specifically demonstrated to achieve remission or cure by inhibiting gene expression using a gene inhibitor. In particular, diseases including cancers and autoimmune diseases have been known to be caused by gene expression in a cell population at a subset level. In such diseases, the use of the carrier of the present invention allows efficient delivery of a gene inhibitor to a target cell, and is thus expected to enhance the effect on remission or treatment and reduce adverse side effects.

The target gene of which the expression is to be inhibited in the present invention may be, for example, but is not limited to, the glyceraldehyde 3-phosphate dehydrogenase (GAPDH) gene, or Foxp3, which is a master gene of Treg cells. In particular, when the target gene is Foxp3, the function of Treg cells can be inhibited, and anti-cancer immune response suppressed by Treg cells can be activated.

In an embodiment, the present invention relates to a carrier for selective delivery to a target cell having multiple types of target receptors on its surface produced by the production method described above.

### EXAMPLES

### 1. Subsets of Cells and Antibody Fragments

The Examples were carried out using the following three sets of subsets of cells and antibody fragments:
(1) a subset of T cells: CD4(+) CD25(+) cells (Treg cells),
   antibody fragments: the cells were targeted using CD4-Fab' and CD25-Fab';
(2) a subset of NK/NKT cells: NK1.1(+) CD27(+) cells (mature NK/NKT cells),
   antibody fragments: the cells were targeted using NK1.1-Fab' and CD27-Fab'; and
(3) a subset of dendritic cells: CD8(+) CD11c(+) cells (CD8+ dendritic cells),
   antibody fragments: the cells were targeted using CD8-Fab' and CD11c-Fab'.

### 2. Preparation of Fab' Fragments

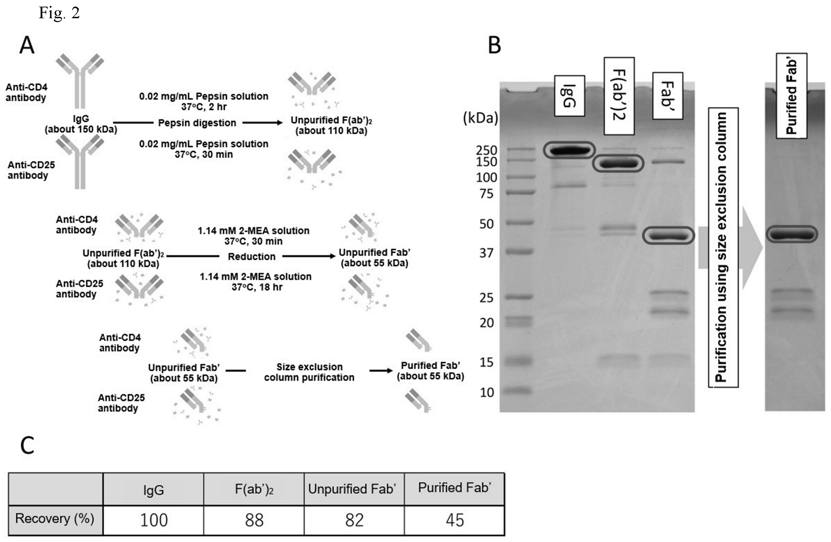

The preparation of Fab' fragments will be described below, taking as an example the preparation of CD4-Fab' fragments (Fig. 2).

Individual types of full-length antibodies were treated with an optimized concentration of pepsin for an optimized digestion time to prepare F(ab')₂ fragments. Then, the F(ab')₂ fragments were reduced with an optimized concentration of 2-mercaptoethylamine (2-MEA) for an optimized reaction time to prepare Fab' fragments. The Fab' fragments were purified by size exclusion chromatography to give Fab' fragments for further experiments.

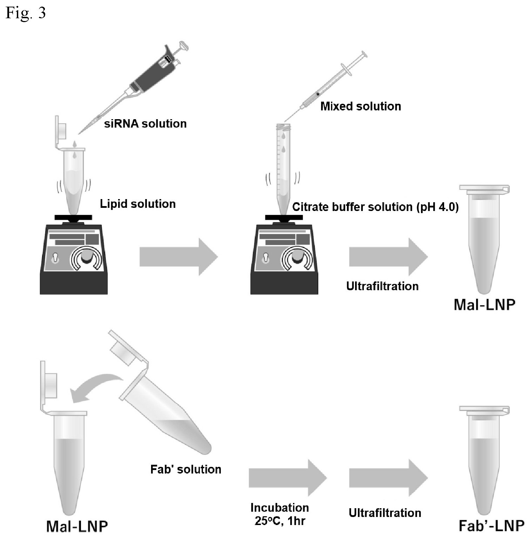

### 3. Preparation of Fab'-modified LNPs (Fig. 3)

LNPs loaded with an siRNA were used as nanoparticles. The lipid composition of the LNPs was CL4H6/cholesterol/DSPC/DMG-PEG2k/DSPE-PEG2k-Mal = 50/10/38/1.5/0.5 (mol ratio). For the preparation of unmodified LNPs without Fab', DSPE-PEG2k was used in place of DSPE-PEG2k-Mal.

The siRNA-loaded LNPs carrying an siRNA against CD45 were prepared by the alcohol dilution method. Each type of Fab' fragment was added to the LNPs at various concentrations to allow the SH groups of the Fab' fragments to react with the maleimide groups on the surface of the LNPs, thereby modifying the surface of the LNPs with the Fab' fragments. DiD was used as a fluorescent tracer to evaluate the uptake of the LNPs by cells.

Each type of Fab' fragment may be represented by the following symbol:
CD4-Fab': Fab' against CD4,
CD25-Fab': Fab' against CD25,
NK1.1-Fab': Fab' against NK1.1,
CD27-Fab': Fab' against CD27,
CD8-Fab': Fab' against CD8, and
CD11c-Fab': Fab' against CD11c.

### 4. Targeting of Subset of Cells (In Vitro)

### (1) Targeting of CD4(+) CD25(+) Cells (Treg Cells) (In Vitro)

CD4-Fab' and CD25-Fab' were employed to identify CD4(+) CD25(+) cells (Treg cells). To determine the modification concentrations of CD4-Fab' and CD25-Fab' on the LNPs, the relationship between the cellular uptake of the LNPs modified with each type of Fab' and the modification concentration of each type of Fab' was examined (Fig. 4). One × 10⁹ DiD-modified LNPs (200 LNPs per cell) were added to mouse spleen cells (5 × 10⁶ cells) and cultured for 2 hours. The spleen cells were collected and fluorescently stained with antibodies against CD4 and CD25 for flow cytometry (FCM) analysis. The result demonstrates that CD4-LNPs were taken up by two types of CD4-expressing cells in a manner depending on the modification concentration of CD4-Fab'. CD25-LNPs were taken up by two types of CD25-expressing cells, and particularly high uptake was observed in CD4(+) CD25(+) cells.

The modification concentration at the point of intersection of the regression line drawn from plots 1 to 4 shown in Table 1 and the regression line drawn from plots 4 to 6 shown in Table 1 was 0.156 µM. Based on this, 0.1 µM (about 0.64-fold the modification concentration at the point of intersection) was selected as a candidate modification concentration at which the single type CD4-Fab' shows no uptake.

**Table 1: CD4-Fab' concentration and the amount of uptake by target cells (CD4(+) CD25(+) cells) (Fig. 4, left)**

| Plot | 1 | 2 | 3 | 4 | 5 | 6 |
|---|---|---|---|---|---|---|
| Concentration (µM) | 0 | 0.05 | 0.1 | 0.15 | 0.2 | 0.3 |
| FI (median) value | 39.6 | 66.0 | 126.3 | 353.3 | 2294.3 | 9110.0 |

The modification concentration at the point of intersection of the regression line drawn from plots 1 to 4 shown in Table 2 and the regression line drawn from plots 4 to 7 shown in Table 2 was 1.28 µM. Based on this, 1 µM (about 0.78-fold the modification concentration at the point of intersection) was selected as a candidate modification concentration at which the single type CD25-Fab' shows no uptake.

**Table 2: CD25-Fab' concentration and the amount of uptake by target cells (CD4(+) CD25(+) cells) (Fig. 4, right)**

| Plot | 1 | 2 | 3 | 4 | 5 | 6 | 7 |
|---|---|---|---|---|---|---|---|
| Concentration (µM) | 0 | 0.5 | 1 | 1.5 | 2 | 3 | 5 |
| FI (median) value | 39.6 | 72.3 | 138.6 | 199.6 | 400.6 | 766.6 | 1306.3 |

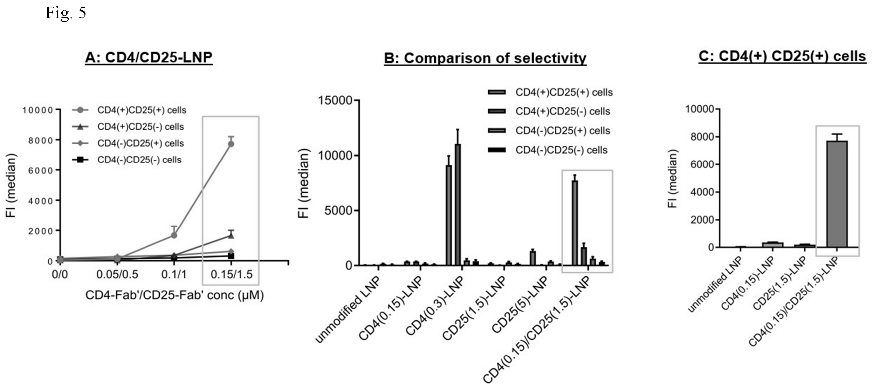

Using the selected candidate concentrations as references, three types of CD4/CD25-LNPs were prepared by setting the modification concentrations of CD4-Fab'/CD25-Fab' to 0.05 µM/0.5 µM, 0.1 µM/1 µM, and 0.15 µM/1.5 µM, respectively. Then, cellular uptake was evaluated in the same manner as above. As a result, the most preferable higher uptake selective to CD4(+) CD25(+) cells was observed at modification concentrations of 0.15 µM/1.5 µM (Fig. 5A). Additionally, the selectivity of CD4/CD25-LNPs was compared with that of CD4-LNPs and CD25-LNPs, revealing that CD4/CD25-LNPs showed higher selectivity to the target subset of CD4(+) CD25(+) cells (Fig. 5B). We have confirmed that the uptake efficiency of the LNPs by the target subset of CD4(+) CD25(+) cells greatly decreased in CD4-LNPs and CD25-LNPs as compared with that of CD4/CD25-LNPs at the same modification concentration (Fig. 5C).

Taken together, we succeeded in the targeting of the subset of CD4(+) CD25(+) cells by controlling the modification concentrations of CD4-Fab' and CD25-Fab' on the surface of the LNPs.

### (2) Targeting of NK1.1(+) CD27(+) Cells (Mature NK/NKT Cells) (In Vitro)

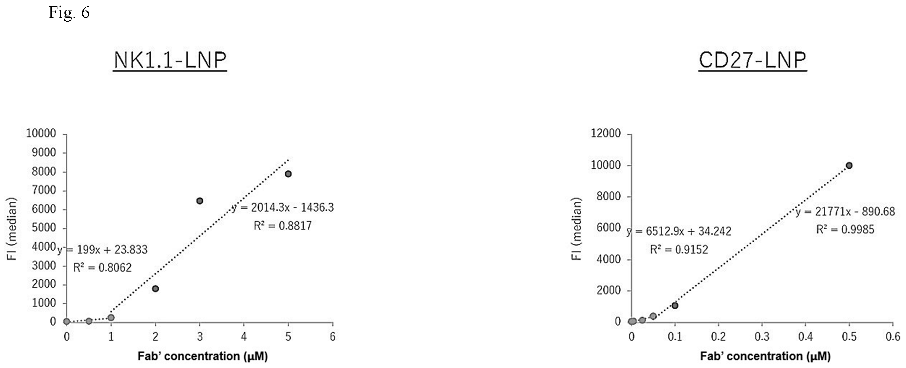

NK1.1-Fab' and CD27-Fab' were employed to identify NK1.1(+) CD27(+) cells (mature NK/NKT cells). To determine the modification concentrations of NK1.1-Fab' and CD27-Fab' on the LNPs, the relationship between the cellular uptake of the LNPs modified with each type of Fab' and the modification concentration of each type of Fab' was examined (Fig. 6). One × 10⁹ DiD-modified LNPs (200 LNPs per cell) were added to mouse spleen cells (5 × 10⁶ cells) and cultured for 2 hours. The spleen cells were collected and fluorescently stained with antibodies against NK1.1 and CD27 for FCM analysis. The result demonstrates that NK1.1-LNPs were taken up by two types of NK1.1-expressing cells in a manner depending on the modification concentration of NK1.1-Fab'. CD27-LNPs were taken up by two types of CD25-expressing cells in a manner depending on the modification concentration of CD27-Fab'.

The modification concentration at the point of intersection of the regression line drawn from plots 1 to 3 shown in Table 3 and the regression line drawn from plots 3 to 8 shown in Table 3 was 0.804 µM. Based on this, 1 µM (about 1.24-fold the modification concentration at the point of intersection) was selected as a candidate modification concentration at which the single type NK1.1-Fab' shows no uptake.

**Table 3: NK1.1-Fab' concentration and the amount of uptake by target cells (NK1.1(+) CD27(+) cells) (Fig. 6, left)**

| Plot | 1 | 2 | 3 | 4 | 5 | 6 |
|---|---|---|---|---|---|---|
| Concentration (µM) | 0 | 0.5 | 1 | 2 | 3 | 5 |
| FI (median) value | 52.0 | 67.0 | 251.0 | 1786.0 | 6470.0 | 7905.0 |

The modification concentration at the point of intersection of the regression line drawn from plots 1 to 4 shown in Table 4 and the regression line drawn from plots 4 to 6 shown in Table 4 was 0.061 µM. Based on this, 0.05 µM (about 0.82-fold the modification concentration at the point of intersection) was selected as a candidate modification concentration at which the single type CD27-Fab' shows no uptake.

**Table 4: CD27-Fab' concentration and the amount of uptake by target cells (NK1.1(+) CD27(+) cells) (Fig. 6, right)**

| Plot | 1 | 2 | 3 | 4 | 5 | 6 |
|---|---|---|---|---|---|---|
| Concentration (µM) | 0 | 0.005 | 0.025 | 0.05 | 0.1 | 0.5 |
| FI (median) value | 59.0 | 76.0 | 131.0 | 392.0 | 1068.0 | 10019.0 |

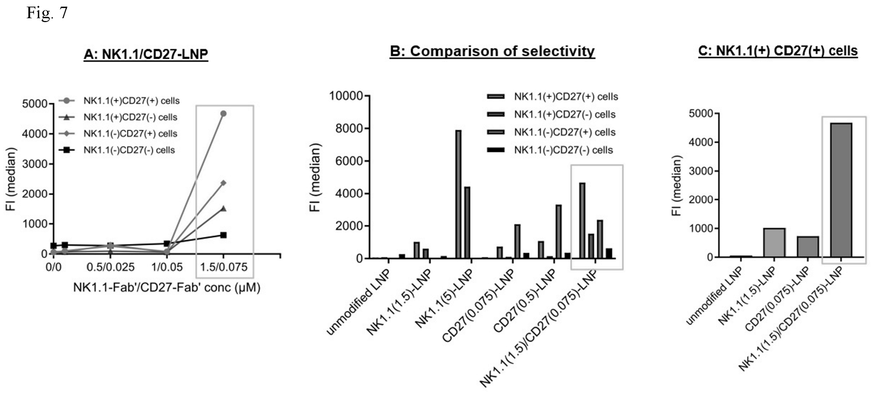

Using the selected candidate concentrations as references, four types of NK1.1/CD27-LNPs were prepared by setting the modification concentrations of NK1.1-Fab'/CD27-Fab' to 0.1 µM/0.005 µM, 0.5 µM/0.025 µM, 1 µM/0.05 µM, and 1.5 µM/0.075 µM, respectively. Then, cellular uptake was evaluated in the same manner as above. As a result, the most preferable higher uptake selective to NK1.1(+) CD27(+) cells was observed at modification concentrations of 1.5 µM/0.075 µM (Fig. 7A). Additionally, the selectivity of NK1.1/CD27-LNPs was compared with that of NK1.1-LNPs and CD27-LNPs, revealing that NK1.1/CD27-LNPs showed higher selectivity to the target subset of NK1.1(+) CD27(+) cells (Fig. 7B). We have confirmed that the uptake efficiency of the LNPs by the target subset of NK1.1(+) CD27(+) cells greatly decreased in NK1.1-LNPs and CD27-LNPs as compared with that of NK1.1/CD27-LNPs at the same modification concentration (Fig. 7C).

Taken together, we succeeded in the targeting of the subset of NK1.1(+) CD27(+) cells by controlling the modification densities of NK1.1-Fab' and CD27-Fab' on the surface of the LNPs.

### (3) Targeting of CD8(+) CD11c(+) Cells (CD8+ Dendritic Cells) (In Vitro)

CD8-Fab' and CD11c-Fab' were employed to identify CD8(+) CD11c(+) cells (CD8+ dendritic cells). To determine the modification concentrations of CD8-Fab' and CD11c-Fab' on the LNPs, the relationship between the cellular uptake of the LNPs modified with each type of Fab' and the modification concentration of each type of Fab' was examined (Fig. 8). One × 10⁹ DiD-modified LNPs (200 LNPs per cell) were added to mouse spleen cells (5 × 10⁶ cells) and cultured for 2 hours. The spleen cells were collected and fluorescently stained with antibodies against CD8 and CD11c for FCM analysis. The result demonstrates that CD8-LNPs were taken up by two types of CD8-expressing cells in a manner depending on the modification concentration of CD8-Fab'. CD11c-LNPs were taken up by two types of CD11c-expressing cells in a manner depending on the modification concentration of CD11c-Fab'.

The modification concentration at the point of intersection of the regression line drawn from plots 1 to 3 shown in Table 5 and the regression line drawn from plots 3 to 6 shown in Table 5 was 0.315 µM. Based on this, 0.25 µM (about 0.79-fold the modification concentration at the point of intersection) was selected as a candidate modification concentration at which the single type CD8-Fab' shows no uptake.

**Table 5: CD8-Fab' concentration and the amount of uptake by target cells (CD8(+) CD11c(+) cells) (Fig. 8, left)**

| Plot | 1 | 2 | 3 | 4 | 5 | 6 |
|---|---|---|---|---|---|---|
| Concentration (µM) | 0 | 0.125 | 0.25 | 0.5 | 1 | 2 |
| FI (median) value | 730.8 | 1154.8 | 2771.8 | 12675.8 | 26795.8 | 80130.8 |

The modification concentration at the point of intersection of the regression line drawn from plots 1 and 2 shown in Table 6 and the regression line drawn from plots 2 to 5 shown in Table 6 was 0.108 µM. Based on this, 0.125 µM (about 1.16-fold the modification concentration at the point of intersection) was selected as a candidate modification concentration at which the single type CD11c-Fab' shows no uptake.

**Table 6: CD11c-Fab' concentration and the amount of uptake by target cells (CD8(+) CD11c(+) cells) (Fig. 8, right)**

| Plot | 1 | 2 | 3 | 4 | 5 |
|---|---|---|---|---|---|
| Concentration (µM) | 0 | 0.125 | 0.25 | 0.5 | 1 |
| FI (median) value | 730.8 | 11539.8 | 75570.8 | 113988.8 | 304715.8 |

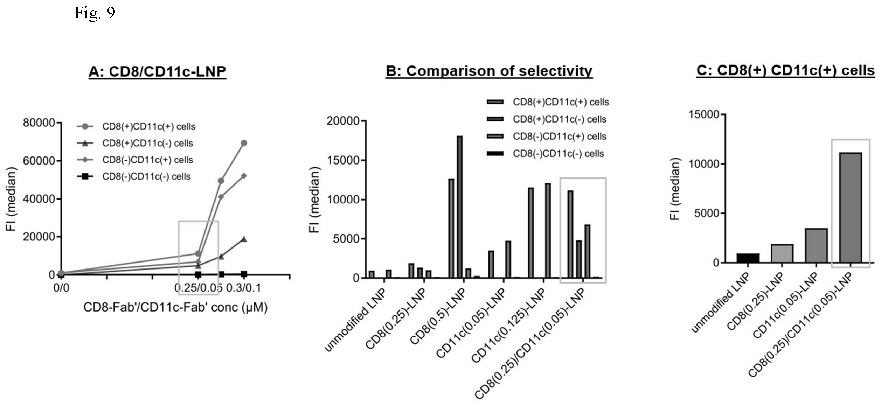

Using the selected candidate concentrations as references, three types of CD8/CD11c-LNPs were prepared by setting the modification concentrations of CD8-Fab'/CD11c-Fab' to 0.25 µM/0.05 µM, 0.25 µM/0.1 µM, and 0.3 µM/0.1 µM, respectively. Then, cellular uptake was evaluated in the same manner as above. As a result, the most preferable higher uptake selective to CD8(+) CD11c(+) cells was observed at modification concentrations of 0.25 µM/0.05 µM (Fig. 9A). Additionally, the selectivity of CD8/CD11c-LNPs was compared with that of CD8-LNPs and CD11c-LNPs, revealing that CD8/CD11c-LNPs showed higher selectivity to the target subset of CD8(+) CD11c(+) cells (Fig. 9B). We have confirmed that the uptake efficiency of the LNPs by the target subset of CD8(+) CD11c(+) cells greatly decreased in CD8-LNPs and CD11c-LNPs as compared with that of CD8/CD11c-LNPs at the same modification concentration (Fig. 9C).

Taken together, we succeeded in the targeting of the subset of CD8(+) CD11c(+) cells by controlling the modification densities of CD8-Fab' and CD11c-Fab' on the surface of the LNPs.

### 5. Influence of Number of Particles on Uptake (In Vitro)

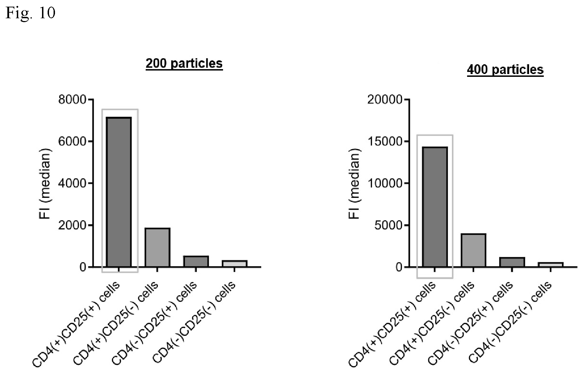

The influence of variation in the number of particles per cell on the cell selectivity was investigated. One × 10⁹ or 2 × 10⁹ DiD-modified LNPs (200 or 400 LNPs per cell) were added to mouse spleen cells (5 × 10⁶ cells) and cultured for 2 hours. The spleen cells were collected and fluorescently stained with antibodies against CD4 and CD25 for FCM analysis. CD4 (0.15)/CD25 (1.5)-LNPs modified with 0.15 µM CD4-Fab' and 1.5 µM CD25-Fab' were used. The result demonstrates that high selectivity to CD4(+) CD25(+) cells was maintained even when the number of particles was doubled, and the FI (median) value on the Y-axis increased, suggesting that the amount of uptake increased with the increase in the number of particles (Fig. 10). Taken together, it was suggested that as long as the modification densities of Fab' fragments per particle are maintained at the same level, variations in the number of particles, i.e., the dosage, may have no influence on the selectivity to a subset of cells.

### 6. In Vivo Evaluation of Selectivity to CD4(+) CD25(+) Cells (Treg Cells)

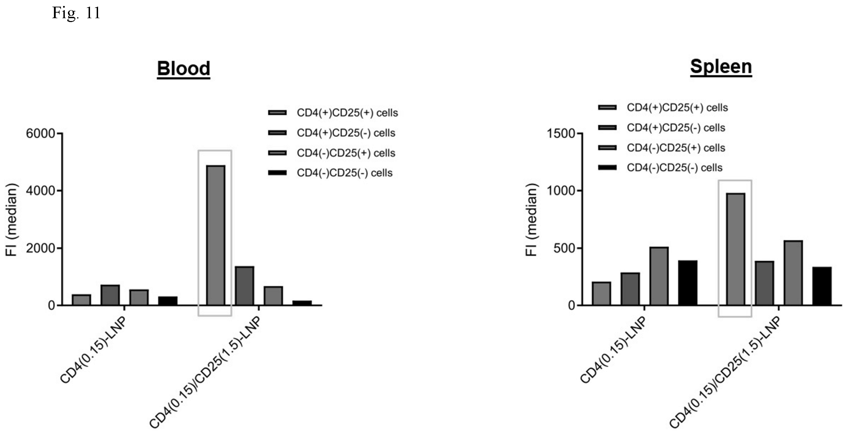

DiD-modified LNPs (50 nmol in terms of the amount of lipids) were administered to mice via the tail vein. One hour after the administration, the blood and spleen were harvested and fluorescently stained with antibodies against CD4 and CD25 for FCM analysis. CD4 (0.15)/CD25 (1.5)-LNPs modified with 0.15 µM CD4-Fab' and 1.5 µM CD25-Fab' were compared with CD4 (0.15)-LNPs modified with 0.15 µM CD4-Fab'. CD4 (0.15)/CD25 (1.5)-LNPs showed higher uptake and higher selectivity to the target CD4(+) CD25(+) cells in the blood and spleen (Fig. 11). On the other hand, CD4 (0.15)-LNPs showed lower uptake by all types of cells and no selectivity to any type of cells. Taken together, we also succeeded in the targeting of the subset of CD4(+) CD25(+) cells in vivo by controlling the modification densities of CD4-Fab' and CD25-Fab' on the surface of the LNPs.

### 7. In Vivo Evaluation of Inhibitory Effect on Expression of Target Gene

### (1) Examination of Cell Selectivity

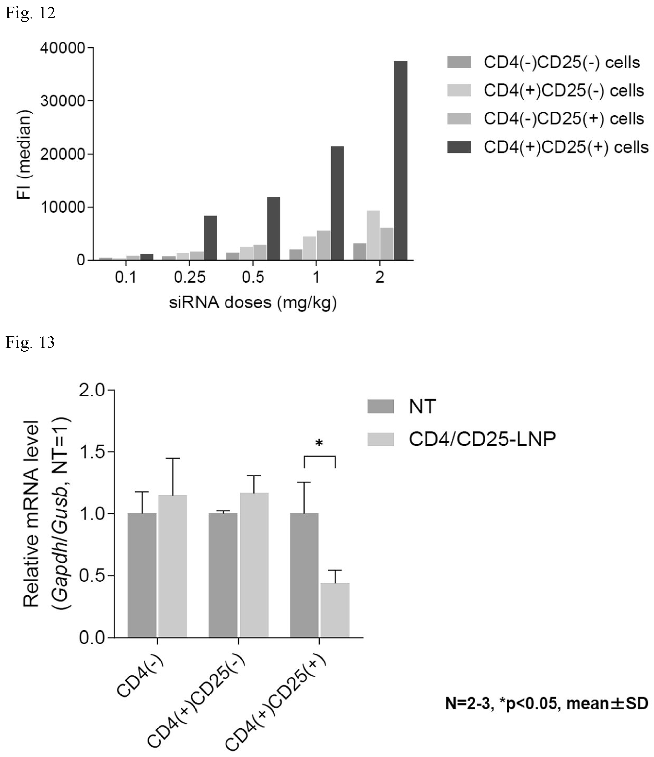

DiD-modified LNPs (0.1, 0.25, 0.5, 1, or 2 mg/kg in terms of the amount of an siRNA) were administered to mice via the tail vein. One hour after the administration, the spleen was harvested and fluorescently stained with antibodies against CD4 and CD25 for FCM analysis. The LNPs used were CD4 (0.15)/CD25 (1.5)-LNPs modified with 0.15 µM CD4-Fab' and 1.5 µM CD25-Fab'. CD4 (0.15)/CD25 (1.5)-LNPs showed high selectivity to the target CD4(+) CD25(+) cells except for the LNPs containing the siRNA at the lowest concentration, 0.1 mg/kg (Fig. 12).

### (2) Examination of Expression Inhibitory Effect (Part 1)

LNPs (20 pmol in terms of the amount of an siRNA; final concentration 40 nM) were added to mouse spleen cells (5 × 10⁶ cells), and the cells were cultured for 24 hours. The spleen cells were collected and divided into CD4(-) cells, CD4(+) CD25(-) cells, and CD4(+) CD25(+) cells using magnetic beads, and RT-qPCR analysis was performed. The result demonstrates that CD4 (0.15)/CD25 (1.5)-LNPs significantly reduced the expression of a target gene only in the CD4(+) CD25(+) cells (Fig. 13).

The siRNA used for the examination of the inhibitory effect on the expression was the commercially available product, Silencer^{™} GAPDH siRNA (mouse) (Thermo Fisher Scientific). Silencer^{™} GAPDH siRNA has been confirmed to inhibit the expression of glyceraldehyde 3-phosphate dehydrogenase (GAPDH). Use of the siRNA for research purposes has been reported in, for example, Nakamura T, et al., Sci Rep 6: 37849, 2016 (Non-Patent Literature 3).

### (3) Examination of Expression Inhibitory Effect (Part 2)

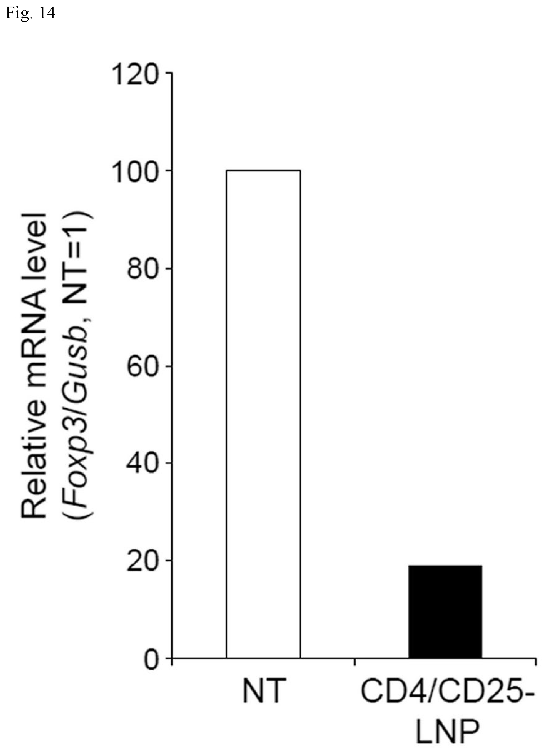

LNPs (200 pmol in terms of the amount of an siRNA; final concentration 400 nM) were added to mouse spleen cells (5 × 10⁶ cells), and the cells were cultured for 24 hours. The spleen cells were collected and divided into CD4(-) cells and CD4(+) cells using magnetic beads, and RT-qPCR analysis of the CD4(+) cells was performed. The result demonstrates that CD4 (0.15)/CD25 (1.5)-LNPs encapsulating an siRNA against Foxp3 reduced the expression of Foxp3 in the CD4(+) cells (Fig. 14). Foxp3 is a master gene of Treg cells. The function of Treg cells can be inhibited by inhibiting Foxp3.

The siRNA used for the examination of the inhibitory effect on the expression was the commercially available product, ON-TARGETplus Mouse Foxp3 siRNA SMARTpool (Dharmacon Inc.).

## Claims

1. A carrier for selective delivery to a target cell having multiple types of target receptors on its surface, the carrier having multiple types of ligands, wherein each type of ligand binds to one of the multiple types of target receptors, wherein the carrier is taken up by the target cell that has all the multiple types of target receptors, but is not taken up by a cell that lacks at least one of the multiple types of target receptors.

2. The carrier according to claim 1, wherein each type of ligand modifies the carrier at a modification concentration at which
(a) any single type of ligand is insufficient to allow the carrier to be taken up by the target cell, but
(b) all the multiple types of ligands together allow the carrier to be taken up by the target cell.

3. The carrier according to claim 2, wherein the multiple types of target receptors are two to five types of receptors.

4. The carrier according to any one of claims 1 to 3, wherein the receptors are antigens, and the ligands are antibodies or antibody fragments.

5. The carrier according to claim 4, wherein the carrier is a lipid nanoparticle, a polymer nanoparticle, a unilamellar liposome, a multilamellar liposome, an O/W type emulsion, a W/O/W type emulsion, a spherical micelle, a wormlike micelle, a lamellar structure, or a mitochondrion.

6. The carrier according to claim 5, wherein the target cell is a subset of immune cells, a subset of cancer cells, or a subset of vascular endothelial cells.

7. The carrier according to claim 6, wherein the subset of immune cells is a subset of T cells, a subset of NK/NKT cells, or a subset of dendritic cells.

8. The carrier according to claim 6, wherein the carrier comprises a nucleic acid, a sugar, a medium molecular weight compound, a low molecular weight compound, or a metal compound.

9. A method for producing a carrier for selective delivery to a target cell having multiple types of target receptors on its surface, the method comprising
(1) determining a modification concentration of each of multiple types of ligands, wherein each type of ligand binds to one of the multiple types of target receptors, wherein the modification concentration of each type of ligand is a concentration at which it is demonstrated that
(a) any single type of ligand is insufficient to allow the carrier to be taken up by the target cell, but
(b) all the multiple types of ligands together allow the carrier to be taken up by the target cell, and
(2) modifying the carrier with the multiple types of ligands each at the determined modification concentration.

10. The production method according to claim 9, wherein the determining the modification concentration of each of the multiple types of ligands is performed by plotting an amount of uptake of the carrier by the target cell against concentrations of each type of ligand, and setting the modification concentration of each type of ligand to 0.4-fold to 2.5-fold the concentration at a point of intersection of a first regression line with a gentle slope drawn from a flat plot in which the amount of uptake of the carrier by the target cell gradually increases or remains unchanged and a second regression line with a steep slope drawn from a plot in which the amount of uptake of the carrier by the target cell rapidly increases.

11. A carrier for selective delivery to a target cell having multiple types of target receptors on its surface produced by the production method according to claim 9 or 10.

12. The carrier according to claim 1, wherein the carrier encapsulates a gene-expression inhibitor, and the carrier is used for inhibition of gene expression.

13. The carrier according to claim 12, wherein the gene expression inhibitor is an siRNA.

14. The carrier according to claim 12, wherein the carrier is used for achieving remission or treatment of a disease caused by overexpression of a gene.

15. The carrier according to claim 14, wherein the disease caused by overexpression of a gene is a cancer or an autoimmune disease.
